# EUROPEAN PATENT APPLICATION

(11) **EP 4 046 578 A1**
(43) Date of publication of application: **24.08.2022**
(21) Application number: 20876688.1
(22) Date of filing: 10.10.2020
(51) Int. Cl.: A61B 17/12

(54) **PLUGGING DEVICE**

(30) Priority: 15.10.2019 CN 201910979284
(71) Applicant: Lifetech Scientific (Shenzhen) Co., Ltd, Shenzhen, Guangdong 518057 (CN)
(72) Inventor: LI, Anning, Shenzhen, Guangdong 518000 (CN); LIU, Jianyong, Shenzhen, Guangdong 518000 (CN)
(74) Representative: Prinz & Partner mbB
(86) International application number: PCT/CN2020/120077
(87) International publication number: WO 2021/073453

(57) **Abstract**

An occluding device (100) includes a sealing disc (110). The sealing disc (110) includes a proximal disc surface (111) and a distal disc surface (112), as well as an edge (113) connecting the proximal disc surface (111) and the distal disc surface (112). The sealing disc (110) is provided with at least one buffer member (114). The at least one buffer member (114) is distributed along the edge (113). The occluding device (100) can reduce the damage inflicted on internal tissues caused by the edge (113) of the sealing disc (110), better protect the internal tissues and reduce the risk of surgery.

## Description

### Technical Field

The disclosure relates to the field of interventional medical instruments, and in particular, to an occluding device for occluding an internal opening.

### Background

In recent years, the application of a split left atrial appendage occluder in interventional therapy or stroke prevention has become more and more popular. Good fixing and sealing properties are major advantages of the split left atrial appendage occluder. However, an internal structure of a cavity of left atrial appendage is not only diverse and complex, and when a selected sealing disc is too small or a release position of an occluding device is too deep, an edge of the sealing disc may directly abut against internal tissues at an opening of the left atrial appendage partially or wholly. Since the edge of the sealing disc has certain hardness and the heart repeatedly undergoes contraction and relaxation, it is likely to cause the edge of the sealing disc to abrade the internal tissues at the opening of the left atrial appendage, which is detrimental for patients.

### Summary

Based on this, it is beneficial to provide a new occluding device in view of the problem that there is a risk of abrading tissues at an internal opening by an edge of a sealing disc.

An occluding device includes a sealing disc. The sealing disc includes a proximal disc surface and a distal disc surface, as well as an edge connecting the proximal disc surface and the distal disc surface. The sealing disc is provided with at least one buffer member. The at least one buffer member is distributed along the edge. The buffer member includes at least one of the following: a film body, a suture line, a metal coil, a bladder member, and a brush.

In one of the embodiments, the occluding device further includes a fixing disc located on one side of the sealing disc. One end of the fixing disc is connected to the sealing disc, and the other end of the fixing disc radiates radially towards a distal end and then extends towards a proximal end.

In one of the embodiments, the at least one buffer member surrounds the edge for at least one circle. Alternatively, when there are a plurality of buffer members, the plurality of buffer members are spaced apart on the edge.

In one of the embodiments, the buffer member is the film body, which is fixed against the edge.

In one of the embodiments, the buffer member is the film body, two borders of the film body along a length direction thereof are respectively fixed to the sealing disc, and a cavity is formed between the film body and the edge.

In one of the embodiments, at least a portion of the proximal disc surface and/or at least a portion of the distal disc surface is coated with a film body.

In one of the embodiments, the edge is a smooth convex edge facing outwards between the proximal disc surface and the distal disc surface.

In one of the embodiments, at least one end of the metal coil is fixed to the sealing disc, and a gap exists between at least a portion of the metal coil and the edge.

In one of the embodiments, one end of the brush is fixed to a proximal or distal end of the sealing disc, and the other end of the brush is a free end.

In one of the embodiments, at least one flow blocking film is arranged on the sealing disc, one end of the brush is fixed to the flow blocking film, and the other end of the brush is a free end.

In the above-described occluding device, at least one buffer member is arranged on a sealing disc and distributed along an edge, so as to prevent the edge of the sealing disc from directly abutting against tissues at an internal opening. Moreover, since the buffer member has a better buffer effect, when the sealing disc interacts with the internal tissues, the damage inflicted on the internal tissues caused by the edge can be reduced, the internal tissues can be better protected, and the risk of surgery can be reduced.

### Brief Description of the Drawings

Fig. 1 is a cross-sectional structure diagram of an occluding device in Embodiment 1;
Fig. 2 is a front structure diagram of a sealing disc in Fig. 1;
Fig. 3 is another structure diagram of an occluding device in Embodiment 1;
Fig. 4 is another cross-sectional structure diagram of a sealing disc of an occluding device in Embodiment 1;
Fig. 5 is another cross-sectional structure diagram of a sealing disc of an occluding device in Embodiment 1;
Fig. 6 is a structure diagram of another sealing disc of an occluding device in Embodiment 1;
Fig. 7 is another cross-sectional structure diagram of an occluding device in Embodiment 1;
Fig. 8 is another cross-sectional structure diagram of a sealing disc of an occluding device in Embodiment 1;
Fig. 9 is another cross-sectional structure diagram of an occluding device in Embodiment 1;
Fig. 10 is another cross-sectional structure diagram of an occluding device in Embodiment 1;
Fig. 11 is a structure diagram of a sealing disc of an occluding device in Embodiment 2;
Fig. 12 is a structure diagram of a sealing disc of an occluding device in Embodiment 3;
Fig. 13 is another structure diagram of a sealing disc of an occluding device in Embodiment 3;
Fig. 14 is a side structure diagram of a sealing disc of an occluding device in Embodiment 5;
Fig. 15 is a structure diagram of a compressed sealing disc of an occluding device in Embodiment 5;
Fig. 16 is another partial structure diagram of a sealing disc of an occluding device in Embodiment 5; and
Fig. 17 is another structure diagram of a sealing disc of an occluding device in Embodiment 5.

### Detailed Description of the Embodiments

In order to make the objects, solutions and advantages clearer, the disclosure will be further described in detail below with reference to the accompanying drawings and embodiments. It should be understood that specific embodiments described herein are illustrative only and are not limiting.

It should be noted that in the field of interventional medical instruments, an end of a medical instrument implanted in a human or animal body that is closer to an operator is generally referred to as a "proximal end", an end that is further away from the operator is referred to as a "distal end", and the "proximal end" and "distal end" of any component of the medical instrument are defined in accordance with this principle. An "axial direction" generally refers to a length direction of the medical instrument when being delivered, a "radial direction" generally refers to a direction of the medical instrument perpendicular to the "axial direction" thereof, and the "axial direction" and "radial direction" of any component of the medical instrument are defined in accordance with this principle.

The solutions of the disclosure will be described below in further detail with reference to specific embodiments.

### Embodiment 1

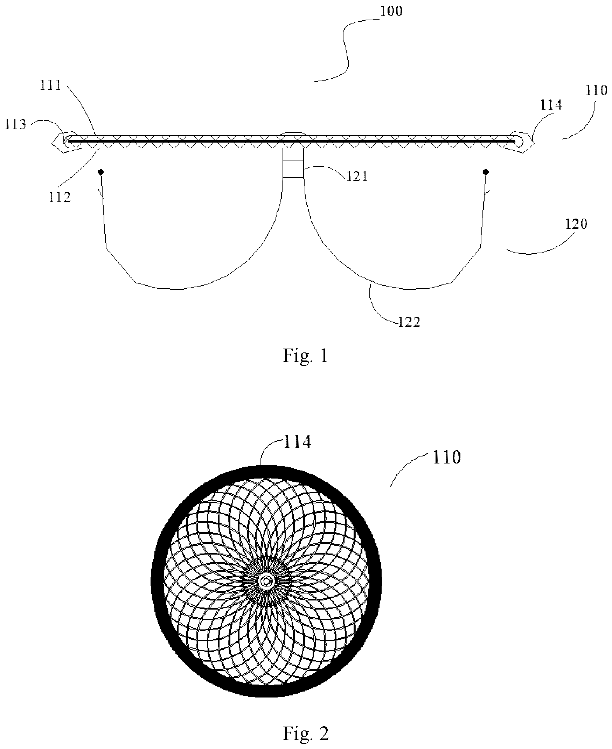

Referring to Figs. 1 and 2, Embodiment 1 provides an occluding device 100 for occluding tissue having an opening in a human or animal body, such as left atrial appendage or foramen ovale. The occluding device 100 includes a sealing disc 110 and a fixing disc 120 located on one side of the sealing disc 110. One end of the fixing disc 120 is directly connected to the sealing disc 110, and the other end of the fixing disc radiates radially towards a distal end and then extends towards a proximal end. The sealing disc 110 includes a proximal disc surface 111 and a distal disc surface 112, as well as an edge 113 connecting the proximal disc surface 111 and the distal disc surface 112. The sealing disc 110 is provided with at least one buffer member 114. These buffer members 114 are all distributed along the edge 113.

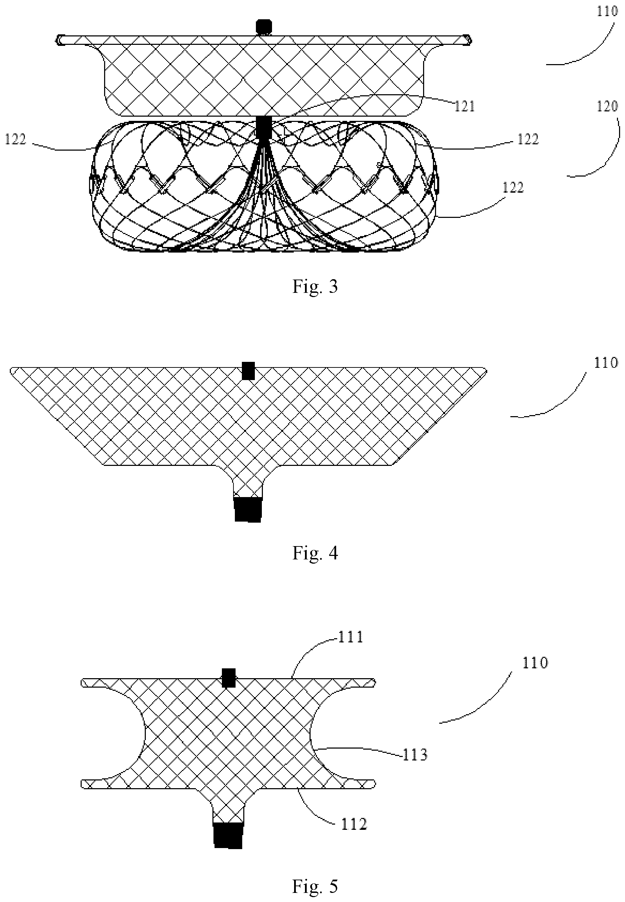

In this embodiment, the fixing disc 120 includes a connecting portion 121 and a plurality of supporting bodies 122. The connecting portion 121 is connected to the distal end of the sealing disc 110. One end of each of the plurality of supporting bodies 122 is connected to the connecting portion 121, and the other end radiates radially from the connecting portion 121 towards the distal end and then extends towards the proximal end. The plurality of supporting bodies 122 cooperate to form a recessed region at the distal end of the fixing disc 120, and free ends of the plurality of supporting bodies 122 cooperate to form a proximal opening at the proximal end of the fixing disc 120. The fixing disc 120 shown in Fig. 1 is heat-set into an umbrella shape by laser cutting of a hollow metal tube. An uncut end of the metal tube serves as the connecting portion 121, and a plurality of cut metal rods serve as the plurality of supporting bodies 122. In other embodiments, as shown in Fig. 3, the fixing disc 120 may be woven into a mesh tube shape from at least one metal wire, and then one end thereof is fixed by closing up a weaving wire through a sleeve, and heat-set into an umbrella shape. The fixing disc 120 also includes a recessed region at the distal end thereof, and a proximal opening at the proximal end thereof. The sleeve is the connecting portion 121 of the fixing disc 120, and a plurality of metal wire segments located between the connecting portion 121 of the fixing disc 120 and the proximal opening are defined as the plurality of supporting bodies 122.

The sealing disc 110 is woven into a mesh tube shape from at least one metal wire, and then both ends thereof are respectively fixed by closing up a weaving wire through a sleeve, and heat-set into a flat disc shape. A distal sleeve of the sealing disc 110 is connected to the connection portion 121, and a proximal sleeve of the sealing disc 110 is also configured to be detachably connected to a delivery apparatus for delivering the occluding device 100.

In other embodiments, the connecting portion 121 of the fixing disc 120 is indirectly connected to the distal end of the sealing disc 110 through at least one connector, which may be, for example, a steel sleeve or a flexible metal wire, etc. In other embodiments, the sealing disc 110 may also be shaped like a plug as shown in Fig. 3, a truncated cone as shown in Fig. 4, an hourglass as shown in Fig. 5, etc. The hourglass-shaped sealing disc 110 has a proximal disc surface 111 and a distal disc surface 112, as well as a curved surface located between the proximal disc surface 111 and the distal disc surface 112 and recessed towards a central axis of the sealing disc 110. The curved surface is the edge 113 of the hourglass-shaped sealing disc 110. The hourglass-shaped sealing disc 110 has better sealing and fixing effects. In other embodiments, the sealing disc 110 having sealing and fixing functions may be used alone as the occluding device 100.

In the present embodiment, the buffer member 114 includes at least one elongated film body which surrounds the edge 113 for at least one circle. The film body may be made of high molecular materials such as polyethylene terephthalate (PET) and poly tetra fluoro ethylene (PTFE), and thus has a good flexibility. The buffer member 114 coated near the edge 113 can reduce the abrasion of the edge 113 to internal tissues during atrial fibrillation and play a buffer role, and the sealing performance of the sealing disc 110 can also be improved. In addition, two borders of the film body along a length direction thereof are respectively fixed to the sealing disc 110, and a cavity is formed between the film body and the edge 113, so that the buffer effect is better. The film body may be fixed to the sealing disc 110 by suturing or heat treatment bonding, or gluing, etc.

In other embodiments, the buffer member 114 includes a plurality of film bodies which surround the edge 113 for at least one circle. The plurality of film bodies may overlap around the edge 113, so as to increase the flexibility of the edge 113 in contact with the internal tissues while providing a better buffer effect and reducing the damage to the internal tissues.

In other embodiments, the buffer member 114 includes at least one elongated film body which surrounds the edge 113 for at least one circle. The two borders of the film body along the length direction thereof are respectively fixed to the sealing disc 110, and the film body is fixed against the edge 113, that is, there is no obvious cavity between the film body and the edge 113.

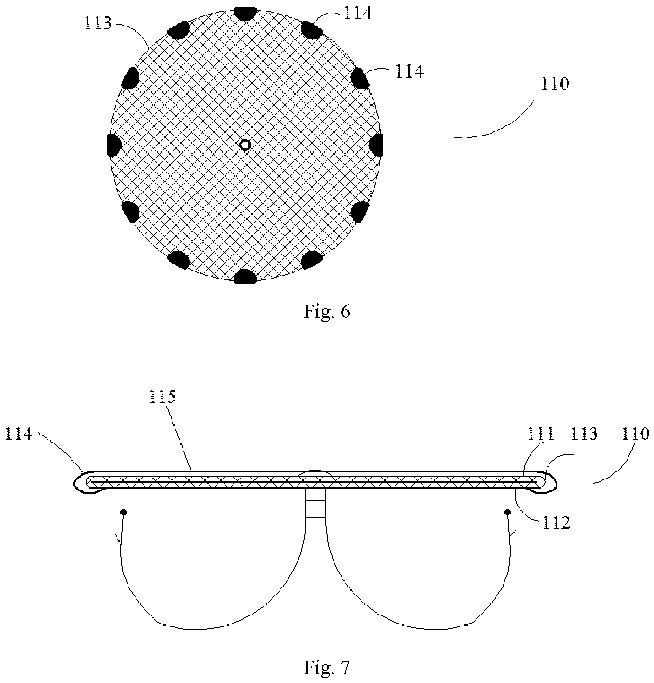

In other embodiments, referring to Fig. 6, the sealing disc 110 is provided with a plurality of buffer members 114. Each buffer member 114 includes a small film body, which is sutured or adhered to the edge 113. All the buffer members 114 are equally or non-equally spaced apart on the edge 113. Such a buffer member 114 creates less resistance when the sealing disc 110 is sheathed than a buffer member 114 that surrounds around the edge 113 for one circle, thereby facilitating the sheathing.

Further, referring to Fig. 7, at least a portion of the proximal disc surface 111 and/or at least a portion of the distal disc surface 112 of the sealing disc 110 is coated with a film body 115. The film body 115 and the film body 114 arranged on the edge 113 may be integrally formed, or may be independent film bodies, which may or may not be connected together by suturing or adhering. Such a sealing disc 110 can not only reduce the damage caused by the friction of the edge 113 against the internal tissues, but also improve the sealing effect of the sealing disc 110 to achieve multiple sealing.

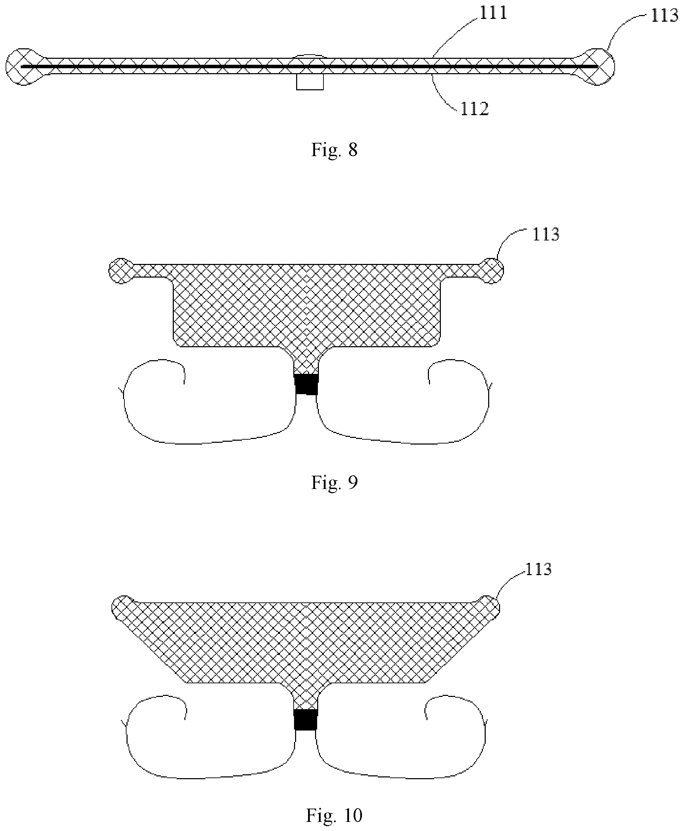

Further, referring to Figs. 8-10, the edge 113 is a smooth convex edge facing outwards between the proximal disc surface 111 and the distal disc surface 112. The smooth convex edge can also reduce the friction of the edge 113 against the internal tissues, thereby cooperating with the buffer member 114 to further reduce the risk of damage to the internal tissues.

The occluding device 100 in the above-described embodiments is constrained to be approximately linear by a cavity of a delivery device such as a sheath when delivered from the outside of a body to the inside of the body. Taking the sheath as an example, when the sheath is withdrawn towards the proximal end during release in the body, the fixing disc 120 at the distal end firstly protrudes from the cavity of the sheath, and the distal end of each supporting body 122 on the fixing disc 120 firstly stretches radially outwards, then bends towards the proximal end, and continues to extend towards the proximal end as the proximal portion of the fixing disc 120 is released from the sheath until it returns to the heat-set shape. The released fixing disc 120 may be fixed to an inner wall of an internal tissue having a cavity such as left atrial appendage, so as to fix the occluding device 100 to the internal tissue. Thereafter, the sheath is further withdrawn towards the proximal end, and the sealing disc 110 protruding from the cavity of the sheath is expanded to the heat-set shape, thereby covering an opening of a part to be occluded and successfully achieving occlusion.

### Embodiment 2

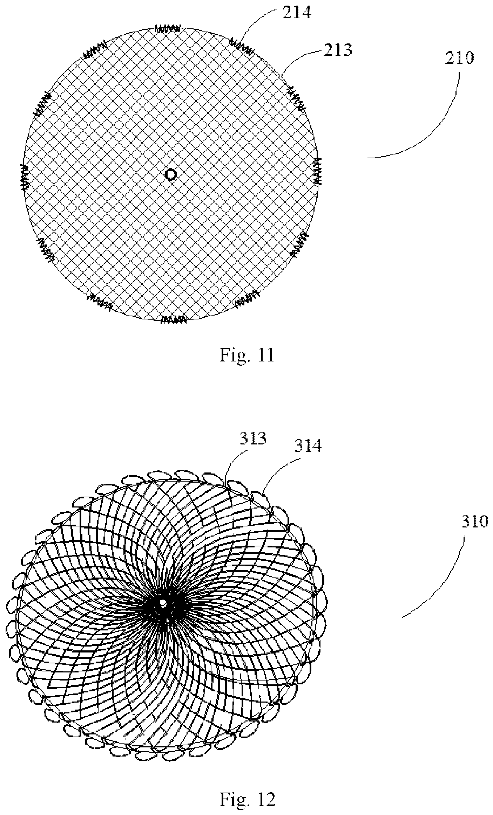

The same portions of the occluding device in Embodiment 2 as those of the occluding device 100 in Embodiment 1 will not be described again here, and the main difference therebetween is that a buffer member 214 on a sealing disc 210 in Embodiment 2 is at least one suture line. Referring to Fig. 11, the buffer member 214 is a plurality of suture lines that are wrapped around different parts of an edge 213, and all the suture lines are equally or non-equally spaced apart on the edge 213. In other embodiments, at least one suture line is wrapped around the edge 213 and surrounds the edge 213 for at least one circle. Such a buffer member 214 can also reduce the abrasion of internal tissues by the edge 213, and play a certain buffer role.

Further, in other embodiments, the buffer member may include the film body 114 in Embodiment 1 and the suture line 214 in Embodiment 2. For example, at least one film body may be arranged on the edge 213 of the sealing disc 210 in the same number and manner as in Embodiment 1, and then the suture line may be wound around the film body and the edge 213 by using a suturing mode in which a suturing needle penetrates through meshes near the film body and the edge 213. Such a buffer member has a better flexibility and cause less abrasion on the internal tissues.

### Embodiment 3

The same portions of the occluding device in Embodiment 3 as those of the occluding device 100 in Embodiment 1 will not be described again here, and the main difference therebetween is that a buffer member on a sealing disc in Embodiment 3 is a plurality of elastic metal coils. Referring to Fig. 12, an edge 313 is provided with a plurality of metal coils 314. Both ends of each metal coil 314 are connected to a sealing disc 310, for example to the edge 313, and a gap exists between each metal coil 314 and the edge 313. The metal coils 314 may be woven when the sealing disc 310 is woven from a metal wire, or may be separately formed and fixed to the vicinity of the edge 313 of the sealing disc 310. Such a buffer member 314 can also reduce the abrasion of internal tissues by the edge 313, and play a certain buffer role.

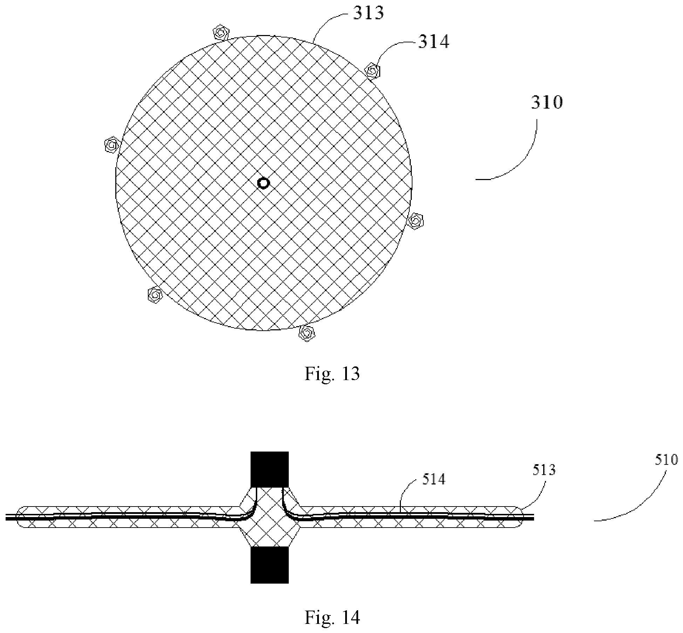

In other embodiments, referring to Fig. 13, the metal coils 314 are in the form of Archimedes screw, one end of the metal coils is fixed to the sealing disc 310, for example to the edge 313, and a plurality of gaps exist between the metal coils 314 and the edge 313, thereby providing a better buffer effect and less abrasion on the internal tissues.

In other embodiments, the edge of the sealing disc 310 in Embodiment 3 may be further coated with some film bodies 114 in Embodiment 1, that is, some or all of the metal coils 314 of the sealing disc 310 in Embodiment 3 are coated with at least one layer of film body 114, and the added film body 114 is arranged against the periphery of the metal coils 314. Thus, the buffer member includes at least the elastic metal coils 314 and the film body 114, which are combined without limitations.

### Embodiment 4

The same portions of the occluding device in Embodiment 4 as those of the occluding device 100 in Embodiment 1 will not be described again here, and the main difference therebetween is that a buffer member in Embodiment 4 includes at least one bladder member. The bladder member may be a closed balloon having a cavity, which may be inflated by injection of liquid, or further may be deflated after slow release of the liquid in the body. When the buffer member is an elongated balloon, the balloon coats at least a portion of an edge along a length direction thereof. In a further embodiment, the length of the balloon just coats the whole edge. When the buffer member is a plurality of balloons, all the balloons are respectively arranged at different parts of the edge and may be fixed to a sealing disc at equal or unequal intervals. Such a buffer member can also reduce the abrasion of internal tissues by the edge, and play a certain buffer role.

The bladder member may be made of materials such as silicone rubber, polyether block polyamide (Pebax), nylon PA11, a mixture of Pebax and nylon PA11, nylon 12, PET, etc.

In use, there is no filler in the bladder member prior to release of the occluding device. After the occluding device is released, a filler is injected into the bladder member through a catheter or the like, thereby inflating the balloon with a good buffer effect. The filler can be liquid, such as physiological saline or another liquid with good biocompatibility. Further, a filler containing an anticoagulant or antiplatelet material may be injected into the bladder member, and the filler may be slowly released from the bladder member into the body, so that a patient does not need to take or takes less anticoagulant medicine after the operation.

### Embodiment 5

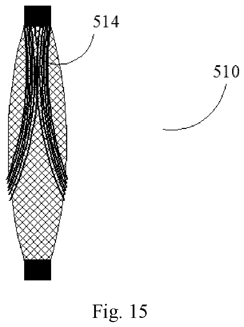

The same portions of the occluding device in Embodiment 5 as those of the occluding device 100 in Embodiment 1 will not be described again here, and the main difference therebetween is that a buffer member in Embodiment 5 includes a plurality of brushes. Referring to Fig. 14, the brushes 514 are in the shape of a thin strip, one end of the brush is fixed to a distal end of a sealing disc 510 (e.g, constrained and fixed by a distal sleeve), the other end is a free end, and a portion between the two ends is received in the sealing disc 510. As shown in Fig. 15, in a compressed state, the free end of the brush 514 may be partially or wholly received in the sealing disc 510. As shown in Fig. 14, in a natural state, the free end of the brush 514 protrudes from the inside of the sealing disc 510 through an edge 513 to the outside. In other embodiments, one end of the brush 514 may be arranged at a proximal end of the sealing disc 510, and the other end may be a free end. Such a buffer member 514 can also reduce the abrasion of internal tissues by the edge 513, and play a certain buffer role.

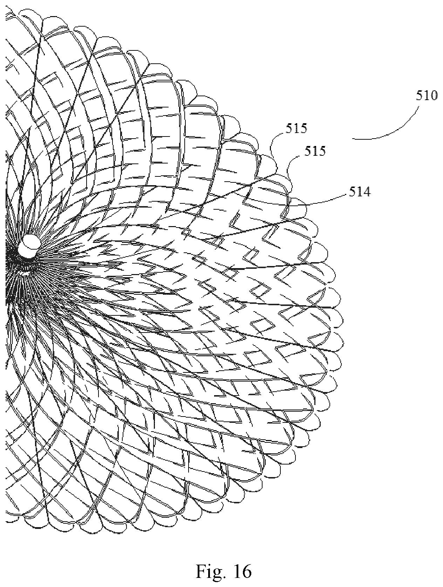

The brushes 514 may be formed of metal wires or high molecular wires with a wire diameter of 0.01 mm to 0.04 mm. The free end of the brush 514 may be further divided into forked portions 515 as shown in Fig. 16, and the forked portions 515 bend towards the sealing disc 510, respectively, thereby not only making a portion of the edge 513 in contact with the internal tissues more flexible, but also avoiding the situation that the brushes 514 cannot come out after directly entering the sealing disc 510, and achieving a limiting effect.

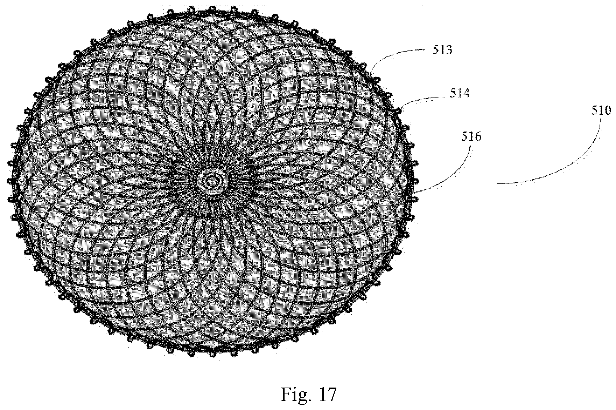

In another embodiment, referring to Fig. 17, at least one flow blocking film 516 is arranged on the sealing disc 510, one end of the brush 514 is fixed to the flow blocking film 516, and the other end of the brush is a free end. For example, more long strips may be cut on border portions of the flow blocking film 516 to serve as the brushes 514. When the flow blocking film 516 is fixed to the sealing disc 510, the portions of the flow blocking film serving as the brushes 514 are located outside the sealing disc 510. Such a flow blocking film 516 not only has a sealing effect, but also can reduce the damage caused by the friction of the edge 513 portion to the internal tissues.

Each feature of the above-described embodiments can be combined in any combination, and, in order to make the description concise, not all the possible combinations of each feature in the above-described embodiments are described. However, as long as there is no contradiction between the combinations of these features, they should be considered as within the scope of the embodiments.

The above-described embodiments express only a few implementations of the disclosure, which are described in greater detail but are not to be construed as limiting the scope of the disclosure. It will be appreciated by those of ordinary skill in the art that numerous variations and modifications may be made to the disclosure without departing from the concept of the disclosure, which fall within the protection scope of the disclosure.

## Claims

1. An occluding device, comprising:
a sealing disc, the sealing disc comprising a proximal disc surface, a distal disc surface, and an edge connecting the proximal disc surface and the distal disc surface, wherein the sealing disc is provided with at least one buffer member, is the buffer member distributed along the edge and the buffer member comprising at least one of the following: a film body, a suture line, a metal coil, a bladder member, and a brush.

2. The occluding device according to claim 1, wherein the occluding device further comprises a fixing disc located on one side of the sealing disc, one end of the fixing disc is connected to the sealing disc, and another end of the fixing disc radiates radially towards a distal end and then extends towards a proximal end.

3. The occluding device according to claim 1 or 2, wherein the at least one buffer member surrounds the edge for at least one circle; or, when there are a plurality of buffer members, the plurality of buffer members are spaced apart on the edge.

4. The occluding device according to claim 3, wherein the buffer member is the film body, and the film body is fixed against the edge.

5. The occluding device according to claim 3, wherein the buffer member is the film body, two borders of the film body along a length direction thereof are respectively fixed to the sealing disc, and a cavity is formed between the film body and the edge.

6. The occluding device according to claim 4 or 5, wherein at least a portion of the proximal disc surface and/or at least a portion of the distal disc surface is coated with a film body.

7. The occluding device according to claim 1, wherein the edge is a smooth convex edge facing outwards between the proximal disc surface and the distal disc surface.

8. The occluding device according to claim 1, wherein at least one end of the metal coil is fixed to the sealing disc, and a gap exists between at least a portion of the metal coil and the edge.

9. The occluding device according to claim 1, wherein one end of the brush is fixed to a proximal or distal end of the sealing disc, and another end of the brush is a free end.

10. The occluding device according to claim 1, wherein at least one flow blocking film is arranged on the sealing disc, one end of the brush is fixed to the flow blocking film, and another end of the brush is a free end.
